# EUROPEAN PATENT APPLICATION

(11) **EP 1 374 877 A1**
(43) Date of publication of application: **02.01.2004**
(21) Application number: 03254127.8
(22) Date of filing: 27.06.2003
(51) Int. Cl.: A61K 35/66, C12N 13/00, A61K 35/72, A23L 1/28, A61K 41/00

(54) **Anti-aging dietary supplements**

(30) Priority: 28.06.2002 US 187114
(71) Applicant: Ultra Biotech Limited, Douglas IM1 1SA, Isle of Man (GB)
(72) Inventor: Cheung, Ling Yuk, Tai Po Industrial Estate, New Territorie (CN)
(74) Representative: Kyle, Diana

(57) **Abstract**

Compositions comprising a plurality of yeast cells, wherein said plurality of yeast cells have been cultured in the presence of an alternating electric field having a specific frequency and a specific field strength for a period of time sufficient to increase the capability of said plurality of yeast cells to have an anti-aging effect. Also included are methods of making such compositions.

## Description

### FIELD OF THE INVENTION

The invention relates to anti-aging compositions that can be taken as dietary supplements. The compositions comprise yeast cells obtainable by growth in electromagnetic fields with specific frequencies and field strengths.

### BACKGROUND OF THE INVENTION

In the past decades, scientists have been trying to understand the biological basis of aging. A multi-disciplinary research area in aging, has developed from fields of biochemistry, molecular biology, and pharmacology. Aging is related with an increase in oxidative damage of cellular DNA, proteins and lipids, which can cause redox imbalance, resulting in the disruption of cellular regulatory processes. The accumulation of autofluorescent, lipoidal, pigmented granules called lipofuscin (LF) in the cytoplasm of neurons parallels cellular aging, see R.S. Sohal, Methods in Enzymology. Vol. 105, pp. 484-487, Academic Press (1984) . The glutathione system, superoxide dismutase (SOD), and catalase (CAT) constitute the main defense system against free radical-induced oxidative damage. Superoxide dismutase (SOD) is an extremely potent antioxidant enzyme that fights cellular damage from reactive free radicals. Increased levels of SOD in the body counters the process of aging. The scavenging of free radicals initiate lipid peroxidation (LPO). Further, monoamine-oxidase type B (MAO-B) levels have been shown to increase in the brain during aging and neurodegeneration. See, R.G. Smith, Current Opinion in Chemical Biology, Vol. 4, pp. 371-376 (2000) .

Vitamin E has been shown to be an anti-oxidant potentially useful in treating aging. However, due to the lack of understanding of maintenance of antioxidant levels, regulation of intracellular antioxidant balance, etc., the uncertainties in using antioxidants as dietary supplements have given anti-aging research great challenges. See B.P. Yu et al., Mechanisms of Ageing and Development. 111, pp. 73-87 (1999). Hormonal intervention using growth hormones, estrogen, Dehydroepiandrosterone and melatonin is also one of the most widely used treatments in anti-aging. However, more and more evidence shows that this method of treatment causes many side effects (B.P. Yu et al., *supra).* Therefore, there is a need for new anti-aging treatments.

### SUMMMARY OF THE INVENTION

This invention is based on the discovery that certain yeast cells can be activated by electromagnetic fields having specific frequencies and field strengths to promote the degradation of aging-related products, for example, LPO, LF and MAO, and the increase in the levels of anti-aging factors such as SOD. Compositions comprising these activated yeast cells can therefore be useful in delaying the process of aging and can be taken as dietary supplements in the form of health drinks or pills.

This invention embraces a composition comprising a plurality of yeast cells that have been cultured in an alternating electric field having a frequency in the range of about 15950 to 16150 MHz, and a field strength in the range of about 100 to 600 mV/cm. In one embodiment, the frequency is in the range of 16000-16100 MHz. In another embodiment, the field strength is in the range of 150-500 mV/cm. The yeast cells are cultured in the alternating electric field for a period of time sufficient to increase the capability of said plurality of yeast cells to lower the levels of LF, LPO or MAO-B in the brain of a mammal as compared to unactivated yeast cells. In another embodiment, the composition comprising the activated yeast cells increases the level of SOD or reduces LPO in the blood of a mammal, as compared to unactivated yeast cells. In one embodiment, the frequency and/or the field strength of the alternating electric field can be altered within the aforementioned ranges during said period of time. In other words, the yeast cells can be exposed to a series of electromagnetic fields. An exemplary period of time is about 40 to 120 hours. In a preferred embodiment, the period of time is 60-90 hours. Included within this invention are also methods of making these compositions.

Yeast cells that can be included in this composition can all be obtained from the China General Microbiological Culture Collection Center ("CGMCC"), a depository recognized under the Budapest Treaty (China Committee for Culture Collection of Microorganisms, Institute of Microbiology, Chinese Academy of Sciences, Haidian, P.O. BOX 2714, Beijing, 100080, China). Useful yeast species include, but are not limited to, *Schizosaccharomyces pombe, Saccharmyces sake, Saccharomyces urarum, Saccharomyces rouxii, Saccharomyces carlsbergensis Hansen*, *Rhodotorula aurantiaca* and *Saccharomyces cerevisiae.* For instance, the yeast cells can be of the strain AS 2.501. In one embodiment, the yeast cells are from the strains selected from the group consisting of AS 2.501, AS2.502, AS2:503, AS2.504, AS2.535, AS2.558, AS2.560, AS2.561 and AS2.562. Other useful yeast species are illustrated in Table 1.

Unless otherwise defined, all technical and scientific terms used herein have the same meaning as commonly understood by one of ordinary skill in the art to which this invention belongs. Exemplary methods and materials are described below, although methods and materials similar or equivalent to those described herein can also be used in the practice or testing of the present invention. All publications and other references mentioned herein are incorporated by reference in their entirety. In case of conflict, the present specification, including definitions, will control. The materials, methods, and examples are illustrative only and not intended to be limiting. Throughout this specification and claims, the word "comprise," or variations such as "comprises" or "comprising" will be understood to imply the inclusion of a stated integer or group of integers but not the exclusion of any other integer or group of integers.

Other features and advantages of the invention will be apparent from the following detailed description, and from the claims.

### BRIEF DESCRIPTION OF THE DRAWINGS

Fig. 1 is a schematic diagram showing an exemplary apparatus for activating yeast cells using electromagnetic fields. 1: yeast culture; 2: container; 3: power supply.
Fig. 2 is a schematic diagram showing an exemplary apparatus for making yeast compositions of the invention. The apparatus comprises a signal generator and interconnected containers 1, 2 and 3.

### DETAILED DESCRIPTION OF THE INVENTION

This invention is based on the discovery that certain yeast strains can be activated by electromagnetic fields ("EMF") having specific frequencies and field strengths to produce agents useful in treating aging. Yeast compositions containing activated yeast cells can be used as a dietary supplement in the form of, e.g., health drinks or pills.

In certain embodiments, the yeast compositions of this invention lower the levels of one or more of LF, LPO and MAO-B in the brain tissue or brain cells of a mammal, such as a human. In other embodiments, the yeast compositions increase the level of SOD, reduce the LPO or both in the blood of a mammal.

Since the activated yeast cells contained in these yeast compositions have been cultured to endure acidic conditions of pH 2.5-4.2, the compositions are stable in the stomach and can pass on to the intestines. Once in the intestines, the yeast cells are ruptured by various digestive enzymes, and the anti-aging agents are released and readily absorbed.

Without being bound by any theory or mechanism, the inventor believes that EMFs activate or enhance the expression of a gene or a set of genes in the yeast cells such that the yeast cells become active or more efficient in performing certain metabolic activities which lead to the production of anti-aging agents.

### I. Yeast Strains Useful in the Invention

The types of yeasts useful in this invention include, but are not limited to, yeasts of the genera *Saccharomyces, Schizosaccharomyces* and *Rhodotorula.*

Exemplary species within the above-listed genera include, but are not limited to, the species illustrated in Table 1. Yeast strains useful in this invention can be obtained from laboratory cultures, or from publically accessible culture depositories, such as CGMCC and the American Type Culture Collection, 10801 University Boulevard, Manassas, VA 20110-2209. Non-limiting examples of useful strains (with the accession numbers of CGMCC) are those illustrated in Table 1. In general, yeast strains preferred in this invention are those used for fermentation in the food and wine industries. As a result, compositions containing these yeast cells are safe for human consumption. Although it is preferred, the preparation of the yeast compositions of this invention is not limited to starting with a pure strain of yeast. A yeast composition of the invention may be produced by culturing a mixture of yeast cells of different species or strains.

**Table 1**

| Exemplary Yeast Strains | | | | |
|---|---|---|---|---|
| | *Saccharomyces cerevisiae* Hansen | | | |
| ACCC2034 | ACCC2035 | ACCC2036 | ACCC2037 | ACCC2038 |
| ACCC2039 | ACCC2040 | ACCC2041 | ACCC2042 | AS2. 1 |
| AS2. 4 | AS2. 11 | AS2. 14 | AS2. 16 | AS2.56 |
| AS2. 69 | AS2. 70 | AS2. 93 | AS2. 98 | AS2. 101 |
| AS2.109 | AS2.110 | AS2.112 | AS2.139 | AS2.173 |
| AS2. 174 | AS2. 182 | AS2. 196 | AS2. 242 | AS2. 336 |
| AS2. 346 | AS2. 369 | AS2. 374 | AS2. 375 | AS2. 379 |
| AS2.380 | AS2.382 | AS2.390 | AS2.393 | AS2.395 |
| AS2.396 | AS2.397 | AS2. 398 | AS2.399 | AS2.400 |
| AS2. 406 | AS2. 408 | AS2. 409 | AS2. 413 | AS2. 414 |
| AS2. 415 | AS2. 416 | AS2. 422 | AS2. 423 | AS2. 430 |
| AS2. 431 | AS2. 432 | AS2. 451 | AS2. 452 | AS2. 453 |
| AS2. 458 | AS2. 460 | AS2. 463 | AS2. 467 | AS2, 486 |
| AS2.501 | AS2. 502 | AS2.503 | AS2.504 | AS2.516 |
| AS2.535 | AS2.536 | AS2.558 | AS2.560 | AS2. 561 |
| AS2.562 | AS2. 576 | AS2.593 | AS2.594 | AS2.614 |
| AS2.620 | AS2.628 | AS2.631 | AS2.666 | AS2.982 |
| AS2. 1190 | AS2. 1364 | AS2. 1396 | IFFI1001 | IFFI1002 |
| IFFI1005 | IFFI1006 | IFFI1008 | IFFI1009 | IFFI1010 |
| IFFI1012 | IFFI1021 | IFFI1027 | IFFI1037 | IFFI1042 |
| IFFI1043 | IFFI1045 | IFFI1048 | IFFI1049 | IFFI1050 |
| IFFI1052 | IFFI1059 | IFFI1060 | IFFI1062 | IFFI1063 |
| IFFI1202. | IFFI1203 | IFFI1206 | 1FFI1209 | IFFI1210 |
| IFFI1211 | IFFI1212 | IFFI1213 | IFFI1214 | IFFI1215 |
| IFFI1220 | IFFI1221 | IFFI1224 | IFFI1247 | IFFI1248 |
| IFFI1251 | IFFI1270 | IFFI1277 | IFFI1287 | IFFI1289 |
| IFFI1290 | IFFI1291 | IFFI1292 | IFFI1293 | IFFI1297 |
| IFFI1300 | IFFI1301 | IFFI1302 | IFFI1307 | IFFI1308 |
| IFFI1309 | IFFI1310 | IFFI1311 | IFFI1331 | IFFI1335 |
| IFFI1336 | IFFI1337 | IFFI1338 | IFFI1339 | IFFI1340 |
| IFFI1345 | IFFI1348 | IFFI1396 | IFFI1397 | IFFI1399 |
| IFFI1411 | IFFI1413 | IFFI1441 | IFFI1443 | |
| | *Saccharomyces cerevisiae* Hansen Var. ellipsoideus (Hansen) Dekker | | | |
| ACCC2043 | AS2.2 | AS2.3 | AS2.8 | AS2.53 |
| AS2.163 | AS2.168 | AS2.483 | AS2.541 | AS2.559 |
| AS2.606 | AS2.607 | AS2.611 | AS2.612 | |
| | *Saccharomyces chevalieri* Guilliermond | | | |
| AS2.131 | AS2.213 | | | |
| | *Saccharomyces delbrueckii* | | | |
| AS2.285 | | | | |
| | *Saccharomyces delbrueckii* Lindner ver. mongolicus (Saito) Lodder et van Rij | | | |
| AS2.209 | AS2.1157 | | | |
| | *Saccharomyces exiguous* Hansen | | | |
| AS2.349 | AS2.1158 | | | |
| | Saccharomyces fermentati (Saito) Lodder et van Rij | | | |
| AS2.286 | AS2.343 | | | |
| | *Saccharomyces logos* van laer et Denamur ex Jorgensen | | | |
| AS2.156 | AS2.327 | AS2.335 | | |
| | *Saccharomyces mellis* (Fabian et Quinet) Lodder et kreger van Rij | | | |
| AS2.195 | | | | |
| | *Saccharomyces mellis* Microellipsoides Osterwalder | | | |
| AS2.699 | | | | |
| | *Saccharomyces oviformis* Osteralder | | | |
| AS2.100 | | | | |
| | *Saccharomyces rosei* (Guilliermond) Lodder et Kreger van Rij | | | |
| AS2.287 | | | | |
| | *Saccharomyces rouxii* Boutroux | | | |
| AS2.178 | AS2.180 | AS2.370 | AS2.371 | |
| | *Saccharomyces sake* Yabe | | | |
| ACCC2045 | | | | |
| | *Candida arborea* | | | |
| AS2.566 | | | | |
| | *Candida lambica* (Lindner et Genoud) van. Uden et Buckley | | | |
| AS2.1182 | | | | |
| | *Candida krusei* (Castellani) Berkhout | | | |
| AS2.1045 | | | | |
| | *Candida lipolytica* (Harrison) Diddens et Lodder | | | |
| AS2.1207 | AS2.1216 | AS2.1220 | AS2.1379 | AS2.1398 |
| AS2.1399 | AS2.1400 | | | |
| | *Candida parapsilosis* (Ashford) Langeron et Talice Var. intermedia Van Rij et Verona | | | |
| AS2.491 | | | | |
| | *Candida parapsilosis* (Ashford) Langeron et Talice | | | |
| AS2.590 | | | | |
| | *Candida pulcherrima* (Lindner) Windisch | | | |
| AS2.492 | | | | |
| | *Candida rugousa* (Anderson) Diddens et Lodder | | | |
| AS2.511 | AS2.1367 | AS2.1369 | AS2.1372 | AS2.1373 |
| AS2.1377 | AS2.1378 | AS2.1384 | | |
| | *Candida tropicalis* (Castellani) Berkhout | | | |
| ACCC2004 | ACCC2005 | ACCC2006 | AS2.164 | AS2.402 |
| AS2.564 | AS2.565 | AS2.567 | AS2.568 | AS2.617 |
| AS2.637 | AS2.1387 | AS2.1397 | | |
| | *Candida utilis* Henneberg Lodder et Kreger Van Rij | | | |
| AS2.120 | AS2.281 | AS2.1180 | | |
| | *Crebrothecium ashbyii* (Guillermond) Routein *(Eremothecium ashbyii* Guilliermond) | | | |
| AS2.481 | AS2.482 | AS2.1197 | | |
| | *Geotrichum candidum* Link | | | |
| ACCC2016 | AS2.361 | AS2.498 | AS2.616 | AS2.1035 |
| AS2.1062 | AS2.1080 | AS2.1132 | AS2.1175 | AS2.1183 |
| | *Hansenula anomala* (Hansen)H et P sydow | | | |
| ACCC2018 | AS2.294 | AS2.295 | AS2.296 | AS2.297 |
| AS2.298 | AS2.299 | AS2.300 | AS2.302 | AS2.338 |
| AS2.339 | AS2.340 | AS2.341 | AS2.470 | AS2.592 |
| AS2.641 | AS2.642 | AS2.782 | AS2.635 | AS2.794 |
| | *Hansenula arabitolgens* Fang | | | |
| AS2.887 | | | | |
| | *Hansenula jadinii* (A. et R Sartory Weill et Meyer) Wickerham | | | |
| ACCC2019 | | | | |
| | *Hansenula saturnus* (Klocker) H et P sydow | | | |
| ACCC2020 | | | | |
| | *Hansenula schneggii* (Weber ) Dekker | | | |
| AS2.304 | | | | |
| | *Hansenula subpelliculosa* Bedford | | | |
| AS2.740 | AS2.760 | AS2.761 | AS2.770 | AS2.783 |
| AS2.790 | AS2.798 | AS2.866 | | |
| | *Kloeckera apiculata* (Reess emend. Klocker) Janke | | | |
| ACCC2022 | ACCC2023 | AS2.197 | AS2.496 | AS2.714 |
| ACCC2021 | AS2.711 | | | |
| | *Lipomycess starkeyi* Lodder et van Rij | | | |
| AS2.1390 | ACCC2024 | | | |
| | *Pichia farinosa* (Lindner) Hansen | | | |
| ACCC2025 | ACCC2026 | AS2.86 | AS2.87 | AS2.705 |
| AS2.803 | | | | |
| | *Pichia membranaefaciens* Hansen | | | |
| ACCC2027 | AS2.89 | AS2.661 | AS2.1039 | |
| | *Rhodosporidium toruloides* Banno | | | |
| ACCC2028 | | | | |
| | *Rhodotorula glutinis* (Fresenius) Harrison | | | |
| AS2.2029 | AS2.280 | ACCC2030 | AS2.102 | AS2.107 |
| AS2.278 | AS2.499 | AS2.694 | AS2.703 | AS2.704 |
| AS2.1146 | | | | |
| | *Rhodotorula minuta* (Saito) Harrison | | | |
| AS2.277 | | | | |
| | *Rhodotorula rubar* (Demme) Lodder | | | |
| AS2.21 | AS2.22 | AS2.103 | AS2.105 | AS2.108 |
| AS2.140 | AS2.166 | AS2.167 | AS2.272 | AS2.279 |
| AS2.282 | ACCC2031 | | | |
| | *Rhodotorula aurantiaca* (Saito) Lodder | | | |
| AS2.102 | AS2.107 | AS2.278 | AS2.499 | AS2.694 |
| AS2.703 | AS2.704 | AS2.1146 | | |
| | *Saccharomyces carlsbergensis* Hansen | | | |
| AS2.113 | ACCC2032 | ACCC2033 | AS2.312 | AS2.116 |
| AS2.118 | AS2.121 | AS2.132 | AS2.162 | AS2.189 |
| AS2.200 | AS2.216 | AS2.265 | AS2.377 | AS2.417 |
| AS2.420 | AS2.440 | AS2.441 | AS2.443 | AS2.444 |
| AS2.459 | AS2.595 | AS2.605 | AS2.638 | AS2.742 |
| AS2.745 | AS2.748 | AS2.1042 | | |
| | *Saccharomyces uvarum* Beijer | | | |
| IFFI1023 | IFFI1032 | IFFI1036 | IFFI1044 | IFFI1072 |
| IFFI1205 | IFFI1207 | | | |
| | *Saccharomyces willianus* Saccardo | | | |
| AS2.5 | AS2.7 | AS2.119 | AS2.152 | AS2.293 |
| AS2.381 | AS2.392 | AS2.434 | AS2.614 | AS2.1189 |
| | *Saccharomyces sp.* | | | |
| AS2.311 | | | | |
| | *Saccharomycodes ludwigii* Hansen | | | |
| ACCC2044 | AS2.243 | AS2.508 | | |
| | *Saccharomycodes sinenses* Yue | | | |
| AS2.1395 | | | | |
| | *Schizosaccharomyces octosporus* Beijerinck | | | |
| ACCC2046 | AS2.1148 | | | |
| | *Schizosaccharomyces pombe* Lindner | | | |
| ACCC2047 | ACCC2048 | AS2.214 | AS2.248 | AS2.249 |
| AS2.255 | AS2.257 | AS2.259 | AS2.260 | AS2.274 |
| AS2.994 | AS2.1043 | AS2.1149 | AS2.1178 | IFFI1056 |
| | *Sporobolomyces roseus* Kluyver et van Niel | | | |
| ACCC2049 | ACCC2050 | AS2.19 | AS2.962 | AS2.1036 |
| ACCC2051 | AS2.261 | AS2.262 | | |
| | *Torulopsis candida* (Saito) Lodder | | | |
| AS2.270 | ACCC2052 | | | |
| | Torulopsis famta (Harrison) Lodder et van Rij | | | |
| ACCC2053 | AS2.685 | | | |
| | *Torulopsis globosa* (Olson et Hammer) Lodder et van Rij | | | |
| ACCC2054 | AS2.202 | | | |
| | *Torulopsis inconspicua* Lodder et Kreger van Rij | | | |
| AS2.75 | | | | |
| | *Trichosporon behrendii* Lodder et Kreger van Rij | | | |
| ACCC2056 | AS2.1193 | | | |
| | *Trichosporon capitatum* Diddens et Lodder | | | |
| ACCC2056 | AS2.1385 | | | |
| | *Trichosporon cutaneum* (de Beurm et al.) Ota | | | |
| ACCC2057 | AS2.25 | AS2.570 | AS2.571 | AS2.1374 |
| | *Wickerhamia fluorescens* (Soneda) Soneda | | | |
| ACCC2058 | AS2.1388 | | | |

### II. Application of Electromagnetic Fields

An electromagnetic field useful in this invention can be generated and applied by various means well known in the art. For instance, the EMF can be generated by applying an alternating electric field or an oscillating magnetic field.

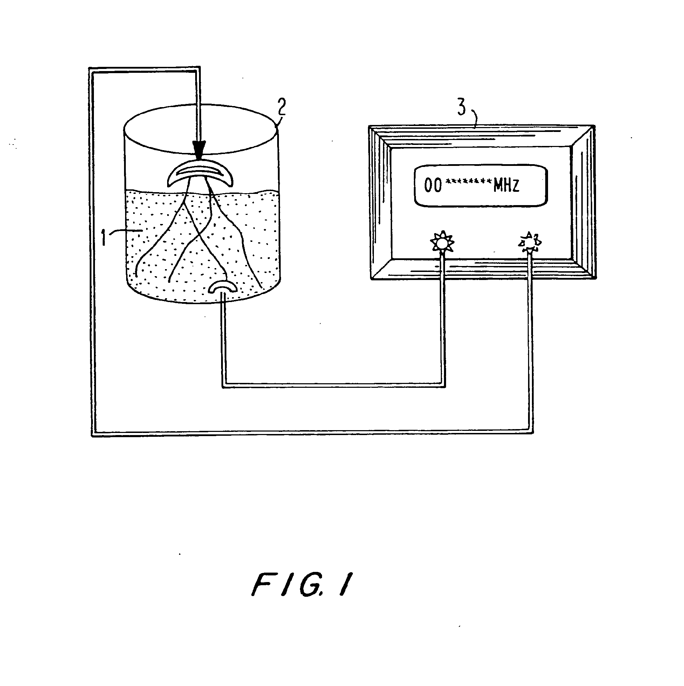

Alternating electric fields can be applied to cell cultures through electrodes in direct contact with the culture medium, or through electromagnetic induction. See, e.g., Fig. 1. Relatively high electric fields in the medium can be generated using a method in which the electrodes are in contact with the medium. Care must be taken to prevent electrolysis at the electrodes from introducing undesired ions into the culture and to prevent contact resistance, bubbles, or other features of electrolysis from dropping the field level below that intended. Electrodes should be matched to their environment, for example, using Ag-AgCl electrodes in solutions rich in chloride ions, and run at as low a voltage as possible. For general review, see Goodman et al., *Effects of EMF on Molecules and Cells,* International Review of Cytology, A Survey of Cell Biology, Vol. 158, Academic Press, 1995.

The EMFs useful in this invention can also be generated by applying an oscillating magnetic field. An oscillating magnetic field can be generated by oscillating electric currents going through Helmholtz coils. Such a magnetic field in turn induces an electric field.

The frequencies of EMFs useful in this invention range from about 15950 MHz to 16150 MHz. Exemplary frequencies include 16016, 16022, 16033, 16051 and 16057 MHz. The field strength of the electric field useful in this invention ranges from about 100 to 600 mV/cm (e.g., 150-200, 170-190, 200-230, 350-360, 370-390, 380-420 or 475-490 mV/cm). Exemplary field strengths include 153, 162, 177, 185, 223, 355, 350, 375, 389, 402 and 485 mV/cm.

When a series of EMFs are applied to a yeast culture, the yeast culture can remain in the same container while the same set of EMF generator and emitters is used to change the frequency and/or field strength. The EMFs in the series can each have a different frequency or a different field strength; or a different frequency and a different field strength. Such frequencies and field strengths are preferably within the above-described ranges. Although any practical number of EMFs can be used in a series, it may be preferred that the yeast culture be exposed to a total of 2, 3, 4, 5, 6, 7, 8, 9 or 10 EMFs in a series.

Although the yeast cells can be activated after even a few hours of culturing in the presence of an EMF, it may be preferred that the activated yeast cells be allowed to multiply and grow in the presence of the EMF(s) for a total of 40-120 hours, preferably 60-90 hours.

Fig. 1 illustrates an exemplary apparatus for generating alternating electric fields. An electric field of a desired frequency and intensity can be generated by an AC source (3) capable of generating an alternating electric field, preferably in a sinusoidal wave form, in the frequency range of 5 to 20,000 MHz. Signal generators capable of generating signals with a narrower frequency range can also be used. If desired, a signal amplifier can also be used to increase the output. The culture container (2) can be made from a non-conductive material, e.g., glass, plastic or ceramic. The cable connecting the culture container (2) and the signal generator (3) is preferably a high frequency coaxial cable with a transmission frequency of at least 20 Ghz. In one embodiment, the transmission frequency is 30 Ghz.

The alternating electric field can be applied to the culture by a variety of means, including placing the yeast culture (1) in close proximity to the signal emitters such as a metal wire or tube capable of transmitting EMFs. The metal wire or tube can be made of red copper, and be placed inside the container (2), reaching as deep as 3-30 cm. For example, if the fluid in the container (2) has a depth of 15-20 cm, 20-30 cm, 30-50 cm, 50-70 cm, 70-100 cm, 100-150 cm or 150-200 cm, the metal wire can be 3-5 cm, 5-7 cm, 7-10 cm, 10-15 cm, 15-20 cm, 20-30 cm and 25-30 cm from the bottom of the container (2), respectively. The number of metal wires/tubes used can be from 1 to 10 (e.g., 2 to 3). It is recommended, though not mandated, that for a culture having a volume up to 10 L, metal wires/tubes having a diameter of 0.5 to 2 mm be used. For a culture having a volume of 10-100 L, metal wires/tubes having a diameter of 3 to 5 mm can be used. For a culture having a volume of 100-1000 L, metal wires/tubes having a diameter of 6 to 15 mm can be used. For a culture having a volume greater than 1000 L, metal wires/tubes having a diameter of 20-25 mm can be used.

In one embodiment, the electric field is applied by electrodes submerged in the culture (1). In this embodiment, one of the electrodes can be a metal plate placed on the bottom of the container (2), and the other electrode can comprise a plurality of electrode wires evenly distributed in the culture (1) so as to achieve even distribution of the electric field energy. The number of electrode wires used depends on the volume of the culture as well as the diameter of the wires.

### III. Culture Media

Culture media useful in this invention contain sources of nutrients that can be assimilated by yeast cells. Complex carbon-containing substances in a suitable form (e.g., carbohydrates such as sucrose, glucose, dextrose, maltose and xylose) can be the carbon sources for yeast cells. The exact quantity of the carbon sources can be adjusted in accordance with the other ingredients of the medium. In general, the amount of carbohydrate varies between about 1% and 10% by weight of the medium, preferably, between about 1% and 5%, and most preferably, the amount of carbohydrate is about 2%. These carbon sources can be used individually or in combination. Amino acid-containing substances such as beef extract and peptone can also be added. In general, the amount of amino acid containing substances varies between about 0.1% and 1% by weight of the medium, and preferably, between about 0.2% and 0.3%. Among the inorganic salts which can be added to a culture medium are the customary salts capable of yielding sodium, potassium, calcium, phosphate, sulfate, carbonate, and like ions. Non-limiting examples of nutrient inorganic salts are (NH₄)₂HPO₄, CaCO₃, KH₂PO₄, K₂ HPO₄, MgSO₄, NaCl, and CaSO₄.

### IV. Electromagnetic Activation of Yeast Cells

To activate or enhance the ability of yeast cells to produce anti-aging agents, these cells can be cultured in an appropriate medium under sterile conditions at 20-35°C (e.g., 28-32°C) for a sufficient amount of time (e.g., 60-90 hours) in an alternating electric field or a series of alternating electric fields as described above.

An exemplary set-up of the culture process is depicted in Fig. 1 (see above). An exemplary culture medium contains the following per 1000 ml of sterile water: 20 g of sucrose, 2 g of peptone, 0.2 g of K₂HPO₄, 0.2 g of KH₂PO₄, 0.2 g of MgSO₄·7H₂O, 0.25 g of NaCl, 0.1 g of CaSO₄·2H₂O, 3 g of CaCO₃·5H₂O, and 1.4 g of beef extract. Yeast cells of the desired strain(s) are then added to the culture medium to form a mixture containing 1X10⁸ cells per 1000 ml of culture medium. The yeast cells can be of any of the strains listed in Table 1. In one embodiment, the strain is *Saccharomyces cerevisiae Hansen* A2.501. The mixture is then added to the apparatus shown in Fig. 1.

The activation process of the yeast cells involves the following steps: (1) maintaining the temperature of the activation apparatus at 20-35°C (e.g., 28-32°C), and culturing the yeast cells for 22-38 hours (e.g., 24 hours); (2) applying an alternating electric field having a frequency of about 16016 MHz and a field strength of 150-200 mV/cm (e.g., about 177 mV/cm) for 14-18 hours (e.g., 15 hours); (3) then applying an alternating electric field having a frequency of about 16022 MHz and a field strength of 170-190 mV/cm (e.g., about 185 mV/cm) for 22-28 hours (e.g., 26 hours); (4) then applying an alternating electric field having a frequency of about 16033 MHz and a field strength of 370-390 mV/cm (e.g., about 389 mV/cm) for 22-28 hours (e.g., 25 hours); (5) then applying an alternating electric field having a frequency of about 16051 MHz and a field strength of 475-490 mV/cm (e.g., about 485 mV/cm) for 10-13 hours (e.g., 12 hours); and (6) then applying an alternating electric field having a frequency of about 16057 MHz and a field strength of 350-360 mV/cm (e.g., about 355 mV/cm) for 11-13 hours (e.g., 12 hours). The activated yeast cells are then recovered from the culture medium by various methods known in the art, dried (e.g., by lyophilization) and stored at 4°C. Preferably, the concentration of the dried yeast cells are no less than 10¹⁰ cells/g.

### V. Acclimatization of Yeast Cells To the Gastric Environment

Because the yeast compositions of this invention must pass through the stomach before reaching the small intestine, where the effective components are released from these yeast cells, it is preferred that these yeast cells be cultured under acidic conditions to acclimatize the cells to the gastric juice. This acclimatization process results in better viability of the yeasts in the acidic gastric environment.

To achieve this, the yeast powder containing activated yeast cells can be mixed with a highly acidic acclimatizing culture medium at 10 g (containing more than 10¹⁰ activated cells per gram) per 1000 ml. The yeast mixture is then cultured first in the presence of an alternating electric field having a frequency of about 16051 MHz and a field strength of 380-420 mV/cm (e.g., about 402 mV/cm) at about 28 to 32°C for 42 to 52 hours (e.g., 48 hours). The resultant yeast cells are further incubated in the presence of an alternating electric field having a frequency of about 16057 MHz and a field strength of 330-360 mV/cm (e.g., about 350 mV/cm) at about 28 to 32°C for 20 to 25 hours (e.g., 22 hours). The resulting acclimatized yeast cells are then either dried and stored in powder form (≥10¹⁰ cells/g) at room temperature or in vacuum at 0-4°C.

An exemplary acclimatizing culture medium is made by mixing 700 ml of fresh pig gastric juice and 300 ml of wild Chinese hawthorn extract. The pH of the acclimatizing culture medium is adjusted to 2.5 with 0.1 M hydrochloric acid and 0.2 M of potassium biphthalate. The fresh pig gastric juice is prepared as follows. At about 4 months of age, newborn Holland white pigs are sacrificed, and the entire contents of their stomachs are retrieved and mixed with 2000 ml of water under sterile conditions. The mixture is then allowed to stand for 6 hours at 4°C under sterile conditions to precipitate food debris. To prepare the wild Chinese hawthorn extract, 500 g of fresh wild Chinese hawthorn is dried under sterile conditions to reduce the water content (≤8%) The dried fruit is then ground (≥20 mesh) and added to 1500 ml of sterile water. The mixture is allowed to stand for 6 hours at 4°C under sterile conditions. The supernatant is collected to be used in the acclimatizing culture medium.

### VI. Manufacture of Yeast Compositions

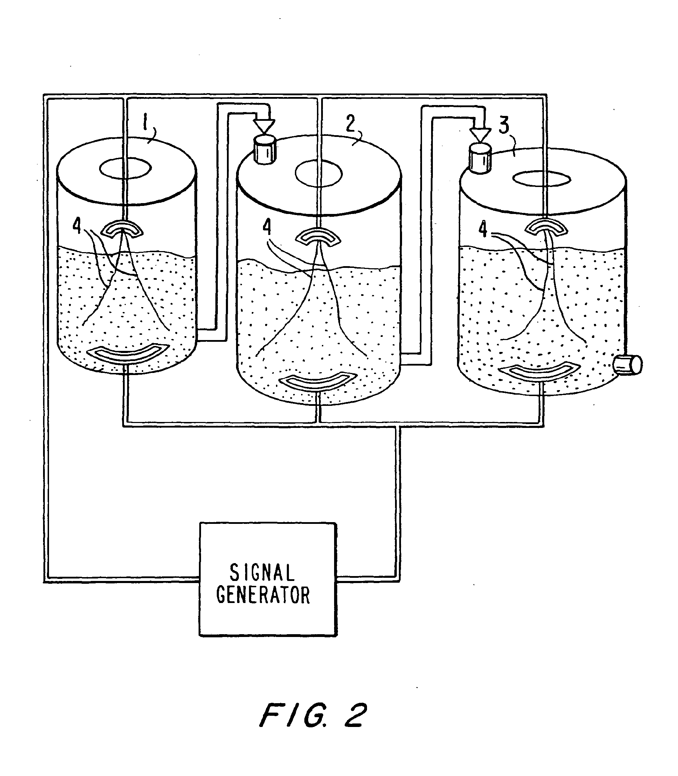

To prepare the yeast compositions of the invention, an apparatus depicted in Fig. 2 or an equivalent thereof can be used. This apparatus includes a first container (1), a second container (2), and a third container (3), each equipped with a pair of electrodes (4). One of the electrodes is a metal plate placed on the bottom of the containers, and the other electrode comprises a plurality of electrode wires evenly distributed in the space within the container to achieve even distribution of the electric field energy. All three pairs of electrodes are connected to a common signal generator.

The culture medium used for this purpose is a mixed fruit extract solution containing the following ingredients per 1000 L: 300 L of wild Chinese hawthorn extract, 300 L of jujube extract, 300 L of fruit extract from *Schisandra chinensis Baill* (wu wei zi), and 100 L of soy bean extracts. To prepare hawthorn, jujube and wu wei zi extracts, the fresh fruits are washed and dried under sterile conditions to reduce the water content to no higher than 8%. One hundred kilograms of the dried fruits are then ground (≥20 mesh) and added to 400 L of sterile water. The mixtures are stirred under sterile conditions at room temperature for twelve hours, and then centrifuged at 1000 rpm to remove insoluble residues. To make the soy bean extract, fresh soy beans are washed and dried under sterile conditions to reduce the water content to no higher than 8%. Thirty kilograms of dried soy beans are then ground into particles of no smaller than 20 mesh, and added to 130 L of sterile water. The mixture is stirred under sterile conditions at room temperature for twelve hours and centrifuged at 1000 rpm to remove insoluble residues. Once the mixed fruit extract solution is prepared, the solution is sterilized at 121°C for 30 minutes, and cooled to 40°C before use.

One thousand grams of the activated yeast powder prepared as described above (Section V, *supra)* is added to 1000 L of the mixed fruit extract solution, and the yeast solution is transferred to the first container (1) shown in Fig. 2. The yeast cells are then cultured in the presence of an alternating electric field having a frequency of about 16051 MHz and a field strength of about 350-400 mV/cm (e.g., 385 mV/cm) at 28-32°C under sterile conditions for 12 hours. The yeast cells are further incubated in an alternating electric field having a frequency of about 16057 MHz and a field strength of 350-400 mV/cm (e.g., about 375 mV/cm). The culturing continues for another 12 hours.

The yeast culture is then transferred from the first container (1) to the second container (2) (if need be, a new batch of yeast culture can be started in the now available first container (1)), and subjected to an alternating electric field having a frequency of about 16051 MHz and a field strength of 150-200 mV/cm (e.g., about 185 mV/cm) for six hours. Subsequently the frequency and field strength of the electric field are changed to about 16057 MHz and 200-230 mV/cm (e.g., about 223 mV/cm), respectively. The culturing continues for another 6 hours.

The yeast culture is then transferred from the second container (2) to the third container (3), and subjected to an alternating electric field having a frequency of about 16051 MHz and a field strength of 150-170 mV/cm (e.g., about 162 mV/cm) for 6 hours. Subsequently the frequency and field strength of the electric field are changed to about 16057 MHz and 145-160 mV/cm (e.g., about 153 mV/cm), respectively. The culturing continues for another 8 hours.

The yeast culture from the third container (3) can then be packaged into vacuum sealed bottles for use as a dietary supplement. The dietary supplement can be taken 3-4 times daily at 30-60 ml each time for a period of three months (10-30 minutes before meals and at bedtime). If desired, the final yeast culture can also be dried within 24 hours and stored in powder form.

In one embodiment, the compositions of the invention can also be administered intravenously or peritoneally in the form of a sterile injectable preparation. Such a sterile preparation is prepared as follows. A sterilized health drink composition is first treated under ultrasound (1000 Hz) for 10 minutes and then centrifuged at 4355 rpm for another 10 minutes. The resulting supernatant is adjusted to pH 7.2-7.4 using 1 M NaOH and subsequently filtered through a membrane (0.22 µm for intravenous injection and 0.45 µm for peritoneal injection) under sterile conditions. The resulting sterile preparation is submerged in a 35-38 °C water bath for 30 minutes before use.

### VII. Examples

The following examples are meant to illustrate the methods and materials of the present invention. Suitable modifications and adaptations of the described conditions and parameters which are obvious to those skilled in the art are within the spirit and scope of the present invention.

The activated yeast compositions used in the following experiments were prepared as described above, using *Saccharomyces cerevisiae Hansen* AS2.501, cultured in the presence of an alternating electric field having the electric field frequency and field strength exemplified in the parentheses following the recommended ranges in Section IV, *supra.* Control yeast compositions were those prepared in the same manner except that the yeast cells were cultured in the absence of EMFs. Unless otherwise indicated, all yeast compositions and the corresponding controls were administered to the animals by intragastric feeding.

### Example 1: LPO, LF and MAO Assays in Mice

A total of 36 NIH mice were selected and separated into three groups (A, B and C) of 12 mice. Each mouse of groups A, B and C was administered daily 3 ml of the activated yeast composition, the control yeast composition and saline, respectively. In addition, each mouse in groups A and B were subcutaneously injected with 0.5 ml of 5% D-galactose daily for 45 days. Each mouse in group C was subcutaneously injected with 0.5 ml of saline for 45 days. All injections were made under sterilized conditions.

After 45 days, the mice were sacrificed. An incision was made in the middle of the brain (excluding the cerebellum). One half of the brain was used for LPO and LF concentration analysis, and the other half was used for MAO-B activity analysis.

### Standard Analysis of LPO Concentrations

Ten times volume of 0.2 M of phosphate-buffered saline (PBS) (pH 7.4) was added to 100-120 mg of brain tissue. The PBS solution was prepared by mixing 0.2 g/L of KCI, 0.2 g/L of KH₂PO₄, 1.56 g/L of NaH₂PO₄·H₂O and 8.0 g/L NaCl and then adjusting the pH to 7.4 by titration with NaHCO₃. The mixture was blended and exposed twice to 12 kHz of ultrasound for 20 seconds with a 30 second interval. Then the mixture was centrifuged at 1000g for 10 minutes at 0°C. The supernatent was placed in a tube containing heparin. 0.3 ml of the solution was taken from the tube and mixed with 4 ml of sulfuric acid and 0.5 ml of phosphotungstic acid, and the mixture was incubated at room temperature for 5 minutes. The mixture was then centrifuged at 3000 rpm for 10 minutes. 2 ml of sulfuric acid and 0.3 ml of phosphotungstic acid were added to the pellet, and the mixture was centrifuged for 10 minutes. Then, 0.3 ml of distilled water was mixed with the pellet.

The concentration of LPO is determined by the concentration of malondialdehyde (MDA), which is the end product from LPO. To determine the MDA concentration, three samples were prepared as illustrated in Table 2.

**Table 2**

| Reagents added | Test sample (ml) | Standard sample (ml) | Blank sample (ml) |
|---|---|---|---|
| brain tissue | 0.3 | | |
| tetraethoxy propane | | 0.3 | |
| methanol | | | 0.3 |
| 0.05 MHC1 | 1 | 1 | |
| 2-Thiobarbituric acid (TBA) | 1 | 1 | 1 |

The three samples were placed in boiling water for 30 minutes and cooled down to room temperature. Then, 4 ml of methanol/1-butanol (15/85 by volume) was added to the samples. The samples underwent rigorous shaking for 45 minutes, and were centrifuged at 4000 rpm for 10 minutes. The layer containing the methanol/1-butanol was extracted and the fluorescence was measured at 532 nm. The MDA concentration was calculated from the formula f/F x 10 mM. F refers to the fluorescence measurement of the standard sample, and f refers to the fluorescence measurement of the test sample.

### Analysis of LF Concentration

100 mg of brain tissue was weighed according to the Sohal method [R.S. Sohal, Methods in Enzymology, Vol.105, pp. 484-487, Academic Press (1984), incorporated herein by reference]. After the addition of 2 ml of an extraction solvent, chloroform-methanol (2:1), the mixture was filtered. The residual material on the filter was washed with the extraction solvent and combined with the filtrate. After adding 5 ml of extraction solvent to the combined filtrate, the solution was subjected to fluorescence measurement with a spectrophotometer. An emission wavelength of 435 nm and an excitation wavelength of 365 nm were used. The LF concentration was determined according to the fluorescence intensity.

### Analysis of MAO Concentration

### 1. Preparation of Enzyme Solution

Ten times volume of 0.2 M of PBS (pH 7.4) was added to about 10 mg of fresh brain tissue. The mixture was blended and exposed twice to 12 kHz of ultrasound for 20 seconds with a 30 second interval. Then, the mixture was centrifuged at 1000g for 10 minutes at 0°C. After removing the precipitate, the supernatent was centrifuged at 17,000 g for 30 minutes at 4°C. The pellet was then mixed with 0.2 M of PBS to form the enzyme solution used in the MAO-B activity assays.

### 2. MAO-B Activity Assay

0.3 ml of 8 mM benzylamine and 2.5 ml of 0.2 M of PBS (pH 7.4) were added to 0.5 ml of the enzyme solution. After placing the solution in a test tube, 3 ml of 0.2 M PBS was added. The solution was incubated at 37°C for three hours, and was shaken every 15 minutes. The reaction was terminated by adding 0.3 ml of perchloric acid (PCA). Then, 4 ml of cyclic pentane was added, and the mixture was centrifuged at 3000 rpm for 10 minutes. The optical density of the solution was determined at 242 nm.

The LPO, LF and MAO-B levels determined using the above methods are illustrated in Table 3.

**Table 3**

| Group | Animal number | LPO (nmol/mg of tissue) | LF fluorescence value | MAO-B (ΔOD /h) |
|---|---|---|---|---|
| A | 12 | 8.79 ± 1.69 | 11.81 ± 1.21 | 0.142 ± 0.006 |
| B | 12 | 10.38 ± 2.32 | 17.86 ± 1.44 | 0.190 ± 0.019 |
| C | 12 | 10.67 ± 1.22 | 18.89 ± 1.43 | 0.220 ± 0.013 |

The experiment shows that for the mice treated with the activated yeast composition (group A), the LF and MAO levels were significantly reduced compared to the mice treated with the control yeast composition or saline (group B or C). In addition, compared to groups B and C, the LPO levels in group A were also generally reduced.

### Example 2: MDA Rat Model Assay

A total of forty 3.5 year old wistar rats were divided into four groups, each containing 10 rats. Groups A, B, C and VE were treated with the activated yeast composition, the control yeast composition, saline and Vitamin E, respectively. Eight 8 month old wistar rats were also selected for group D, which were used as a junior control group.

Groups A, B and VE were administered daily 3 ml/kg (body weight) of the activated yeast composition, 3 ml/kg of the control yeast composition and 0.25 mg/kg of Vitamin E, respectively. Groups C and D were both treated with 3 ml/kg of saline daily. After three months of treatment, blood samples were taken from the heart of the rats and placed in a test tube with heparin. The test tube was then incubated at 37°C for 10 minutes and centrifuged at 2500 rpm for 10 minutes. 4 ml of sulfuric acid and 0.5 ml of phosphotungstic acid were added to the serum and left at room temperature for 5 minutes after mixing. The mixture was then centrifuged at 3000 rpm for 10 minutes. 2 ml of sulfuric acid and 0.3 ml of phosphotungstic acid were added to the precipitate. Finally, the mixture was used to determine the MDA concentration as described previously. The results are illustrated in Table 4.

**Table 4**

| Group | Animal number | MDA (µM) |
|---|---|---|
| A | 10 | 3.12 ± 0.67 |
| B | 10 | 8.35 ± 3.56 |
| C | 10 | 8.58 ± 3.12 |
| D | 8 | 5.12 ± 1.43 |
| VE | 10 | 4.36 ±0.78 |

In this experiment, for the junior rat control group and rats treated with Vitamin E, there was reduced lipid peroxidation. For rats treated with the activated yeast composition (group A), there was significantly reduced lipid peroxidation compared to the rats treated with the control yeast composition or saline (group B or C). Further, the lipid peroxidation in group A was even lower than the lipid peroxidation in the group treated with Vitamin E.

### Example 3: Increased Levels of SOD in Red Blood Cells in Mice

Xanthine oxidase catalyzes the oxidation of xanthine or hypoxanthine into uric acid and oxygen free radicals. The oxygen free radicals react with 3-amino phthalhydrazide to produce a transition state intermediate. When the intermediate reverts back to the basic energy state, it irradiates energy through light emission. As SOD can eliminate the oxygen free radicals, it can suppress the light emission of 3-amino phthalhydrazide. Therefore, the activity of SOD can be determined by the decrease in light emission.

A total of 36 male mice were separated into four groups. Each group contained 9 mice. Groups A and B were administered daily 3 ml/kg (body weight) of the activated yeast composition and the control yeast composition, respectively. Groups C and D were treated with 3 ml/kg (body weight) of saline daily. After 10 days of treatment, Groups A, B and C were subjected to an O₃ environment (0.9 ppm) for 10 days. On day 21, peripheral blood was taken from the mice to determine SOD activity.

10 µl of blood and 240 µl of distilled water were mixed in a test tube. 1-3 µl of anticoagulant was injected to the bottom of the test tube. Then, 5-10 µl of xanthine oxidase, 490 µl of hypoxanthine and 490 µl of 3-amino phthalhydrazide were added to the test tube. A blank sample with 250 µl of distilled water, 1 µl of EDTA, 5-10 µl of xanthine oxidase, 490 µl of hypoxanthine and 490 µl of 3-amino phthalhydrazide was also prepared. After 1 minute of incubation, the peak instantaneous light emission reading was used to calculate the decrease in light emission. The decrease in light emission was determined by comparing the light emission of the test sample to the blank sample as illustrated in Table 5. From the light emission values, the relative SOD activity level in the different groups were compared.

**Table 5**

| Group | Animal number | Light emission (%) |
|---|---|---|
| A | 9 | 44.34 ± 1.38 |
| B | 9 | 58.28 ± 1.69 |
| C | 9 | 67.58 ± 1.45 |
| D | 9 | 52.24 ± 1.12 |

This experiment shows that after 10 days of ozone stress, mice treated with the activated yeast composition (group A) showed a higher level of SOD activity as represented by a smaller percentage of light emission than mice treated with the control yeast composition or saline (groups B and C). Mice from group A and B both showed an increase in SOD activity after ozone stress although such increase seemed to be more significant in mice treated with the activated yeast composition (group A). For mice treated with neither activated nor control yeast composition (group C or D), the presence of ozone stress (group C) seemed to reduce the ability of SOD activation when compared to mice that did not receive ozone stress (group D). Therefore, the activated yeast composition induces SOD activation in the presence of a free radical generator such as ozone.

While a number of embodiments of this invention have been set forth, it is apparent that the basic constructions may be altered to provide other embodiments which utilize the compositions and methods of this invention.

## Claims

1. A composition comprising a plurality of yeast cells, wherein said plurality of yeast cells are **characterized by** an increase in their ability to reduce the level of lipofuscin or monoamine-oxidase type B in the brain of a mammal as a result of having been cultured in the presence of an alternating electric field having a frequency in the range of 15950 to 16150 MHz and a field strength in the range of 100 to 600 mV/cm, as compared to yeast cells not having been so cultured.

2. A composition comprising a plurality of yeast cells, wherein said plurality of yeast cells are **characterized by** an increase in their capability to increase the level of superoxide dismutase or reduce lipid peroxidation in the blood of a mammal as a result of having been cultured in the presence of an alternating electric field having a frequency in the range of 15950 to 16150 MHz and a field strength in the range of 100 to 600 mV/cm, as compared to yeast cells not having been so cultured.

3. The composition of claim 1 or 2, wherein the yeast cells are further **characterized by** an increase in their capability to reduce lipid peroxidation in the brain of a mammal as compared to yeast cells not having been so cultured.

4. The composition of any of claims 1 to 3, wherein the range of the frequency is 16000-16100 MHz.

5. The composition of any of claims 1 to 4, wherein the range of the field strength is 150-500 mV/cm.

6. The composition of any of claims 1 to 5, wherein said yeast cells are of the species selected from the group consisting *of Saccharomyces sp, Schizosaccharomyces pomne Lindner. Saccharmyces sake Yabe, Saccharomyces urarum Beijer, Saccharomyces rouxii Boutroux, Saccharomyces cerevisiae Hansen Var. ellipsoideus, Saccharomyces carlsbergensis Hansen, Rhodotorula aurantiaca Ladder and Saccharomyces cerevisiae Hansen.*

7. The composition of claim 1 or 2, wherein said yeast cells are of the strain deposited at the China General Microbiological Culture Collection Center with an accession number selected from the group consisting of AS 2.501, AS2.502, AS2.503, AS2.504, AS2.535, AS2.558, AS2.560, AS2.561 and AS2.562.

8. The composition of claim 7, wherein the strain is AS2.501.

9. The composition of any of claims 1 to 8, wherein the composition is in the form of a tablet, powder or healthdrink.

10. The composition of claim 9, wherein the composition is in the form of a healthdrink.

11. A method of preparing a yeast composition, comprising culturing a plurality of yeast cells in the presence of an alternating electric field having a frequency in the range of 15950 to 16150 MHz and a field strength in the range of 100 to 600 mV/cm, wherein said plurality of yeast cells are **characterized by** an increase in their ability to reduce the level of lipofuscin or monoamine-oxidase type B in the brain of a mammal as a result of said culturing as compared to yeast cells not having been so cultured.
